Europäisches Patentamt

European Patent Office

Office européen des brevets

(19)

(11) Publication number: 0 153 517

A1

# (12) EUROPEAN PATENT APPLICATION

(21) Application number: 84301344.2

(22) Date of filing: 01.03.84

(51) Int. Cl.⁴: **C 07 C 1/04**
**B 01 J 29/06**

(43) Date of publication of application:
04.09.85 Bulletin 85/36

(84) Designated Contracting States:
DE FR GB IT NL

(71) Applicant: THE STANDARD OIL COMPANY
Midland Building
Cleveland, Ohio 44115(US)

(72) Inventor: Desmond, Michael Joseph
2316 Westminster Road
Cleveland Heights Ohio 44118(US)

(72) Inventor: Pepera, Marc Anthony
7304 Honeydale Drive
Northfield Center Ohio 44067(US)

(74) Representative: Smith, Sydney et al,
Elkington and Fife High Holborn House 52/54 High
Holborn
London WC1V 6SH(GB)

(54) Novel catalysts and their preparation and process for the production of liquid paraffins.

(57) Catalysts comprising a medium to large port zeolite modified with a reducible metal salt of an element selected from the group consisting of Group VIII, IB or IIB elements and, a Fischer-Tropsch catalyst wherein the ratio of zeolite to Fischer-Tropsch component is from 0.1 to 50:1 can be employed to produce liquid paraffins from synthesis gas. A process is employed which includes the step of contacting synthesis gas having a ratio of hydrogen to carbon oxides of from 0.1 to 10.0:1 over the foregoing catalyst at a reaction temperature of from 100°C to 500°C and at a pressure of from one atmosphere (0.1 MPa) to 200 atmospheres (20 MPa) and at a gas hourly spaced velocity of from 10 to 100,000. A method is also provided for the preparation of the combination catalyst. The liquid paraffin product produced is highly branched, relatively free from olefins and totally free from detectable oxygenates.

0153517

# NOVEL CATALYSTS AND THEIR PREPARATION AND
## PROCESS FOR THE PRODUCTION OF LIQUID PARAFFINS

The present invention is directed toward the production of liquid paraffins from synthesis gas. The catalysts and process disclosed are particularly suited for the production of highly branched products with little to no unsaturates and no detectable amounts of oxygenates. Zeolites and Fischer-Tropsch catalysts are employed, both of which have been used extensively for the production of hydrocarbon fuels. A method for the synthesis of the catalysts is also provided.

The conversion of synthesis gas via Fischer-Tropsch catalysts is a well known process. Such catalysts generally produce liquid products which include components such as organic acids, aldehydes, alcohols and olefins which are undesirable for use as fuels. It is also known to employ zeolite catalysts for the conversion of various organic compounds. For example, U.S. Pats. No. 4,276,438 and 4,278,827 disclose processes for the conversion of aromatic compounds to dialkylbenzene compounds. Each process requires a particular type of zeolite catalyst which has been modified with either a Group IB or a Group IVB metal.

U.S. Pat. No. 3,775,501 discloses a process for the production of aromatics from hydrocarbons and air over a zeolite catalyst. U.S. Pat. No. 3,271,418 discloses a process for cracking petroleum gas oil to produce high octane gasoline over a zeolite catalyst where the original alkali metal has been replaced by other cations.

Others have disclosed the use of Fischer-Tropsch catalysts with zeolites in organic synthesis. U.S. Pat. No. 4,279,830, for instance, discloses a two reactor process for converting synthesis gas to oxygenates and hydrocarbons by reducing CO in the first reactor over a Fischer-Tropsch catalyst, where a zeolite is optionally present, followed by treatment in the second reactor over a zeolite catalyst.

U.S. Pat. No. 4,269,783 discloses a process for the conversion of synthesis gases to hydrocarbon mixtures such as olefinic naphtha with an aromatic content of less than 20 weight percent. The olefin plus aromatic content of such products comprises at least 50 weight percent. The zeolite component of the catalyst combination comprises not more than 0.5 weight percent alkali metal.

Lastly, U.S. Pat. No. 4,086,262 discloses a method for the conversion of synthesis gas to hydrocarbon mixtures rich in aromatics. A heterogeneous catalyst comprising zeolites and Fischer-Tropsch synthesis catalysts is employed.

Thus, while the art provides catalysts and processes for the conversion of synthesis gases and other organic feedstocks to various hydrocarbons over mixtures of zeolite and Fischer-Tropsch catalysts, it has not disclosed the specific production of liquid paraffins in high yields at relatively low temperatures and with no detectable amounts of oxygenates and unsaturates.

DISCLOSURE OF INVENTION

It is therefore an object of the present invention to provide a process for producing highly branched liquid paraffins from synthesis gas.

It is another object of the present invention to provide a novel catalyst for converting synthesis gas to highly branched liquid paraffins.

It is still another object of the present invention to provide a method for the preparation of a combination catalyst for the conversion of synthesis gas to highly branched paraffins.

These and other objects, together with the advantages thereof over the prior art, which shall become apparent from the specification which follows, are accomplished by our invention as hereinafter described and claimed.

The catalyst of the present invention comprises a medium to large port zeolite modified with a reducible metal salt of an element selected from the group consisting of Group VIII, IB or IIB elements and, a Fischer-Tropsch catalyst wherein the ratio of zeolite to Fischer-Tropsch component is from about 0.1 to 50:1.

The process of the present invention produces liquid paraffins from synthesis gas and includes the step of contacting synthesis gas having a ratio of hydrogen to carbon oxides of from about 0.1 to 10.0:1 over a combination catalyst at a reaction temperature of from about 100° C to 500° C and at a pressure of from about one atmosphere (0.1 MPa) to about 200 atmospheres (20 MPa) and at a gas hourly space velocity of from about 10 to 100,000. The combination catalyst itself comprises a medium to large port zeolite modified by a reducible metal salt of a Group VIII, IB or IIB element and, a Fischer-Tropsch catalyst wherein the ratio of zeolite to Fischer-Tropsch component is from about 0.1 to 50:1.

A method is also provided for the preparation of a combination catalyst for the conversion of synthesis gas to liquid paraffins. It includes the steps of modifying a medium to large port zeolite with a metal salt of an element selected from the group consisting of Group VIII, IB and IIB and combining the modified zeolite with a Fischer-Tropsch catalyst in a ratio of from about 0.1 to 50:1.

The method can include as further steps the calcining of the modified zeolite at temperatures ranging from about 100° to about 800° C for at least one hour, prior to the combining of the zeolite and Fischer-Tropsch components, and reducing the combination catalyst with $H_2$ and/or CO at a temperature of from about 100° C to 600° C, at $H_2$ and/or CO partial pressures ranging from 0.001 MPa to about 5.07 MPa and for a period of time of from about one to 100 hours prior to employing the catalyst for the conversion of synthesis gas.

The catalyst and process for its use results in a high yield of low boiling point highly branched paraffins. The process is run at relatively low temperatures and olefins and oxygenates are essentially eliminated unlike the existing art where these are often produced.

## PREFERRED MODE FOR CARRYING OUT THE INVENTION

The catalyst of the present invention comprises a mixture or combination of a Fischer-Tropsch catalyst and a medium or large port zeolite which has been modified with a reducible metal salt of a Group VIII, IB or IIB element.

The zeolites capable of use in this invention include medium port zeolites and large port zeolites. Examples of medium port zeolites include ferrierite, chabazite, erionite, ZSM-5 (U.S. Pat. No. 3,702,886) and ZSM-11 (U.S. Pat. No. 3,709,979). Examples of large port zeolites include mordenite, zeolite X (U.S. Pat. No. 2,882,244) and zeolite Y (U.S. Pat. No. 3,130,007). The foregoing list is exemplary only and is not intended to be limiting. A more complete description of the man-made zeolites can be found by referring to the cited patents, the subject matter of which is hereby incorporated by reference. Pore opening diameter of such zeolites range from about 4 Å up to about 10 Å.

The zeolites may be used in a variety of cation exchange forms, preferably where the cations are $NH_4^+$, $H^+$, $Na^+$, $Li^+$, $K^+$, $Cs^+$, $Rb^+$, $Ag^+$ and other monovalent cations. Zeolites with divalent and trivalent metals as cations are less preferred, but still are possible suitable forms. The zeolites are then modified by exchange and/or impregnation with metal salts of Groups VIII, IB or IIB of the Periodic Table. Introduction of zero valent metals, such as by vapor deposition, is also a suitable way of introducing the metal into/onto the zeolite. Metal concentration in the zeolite can range from 0.01 to 20 weight percent and preferably is from 0.1 to 10 weight percent.

The metal loaded zeolite is subsequently dried and calcined at temperatures between 100 and 800° C and preferably from 300 to 600° C. The calcined zeolite is then combined with a supported or unsupported Fischer-Tropsch catalyst, which includes any such catalyst known in the art, either as an intimate mixture or as separate particles. The Fischer-Tropsch catalysts include all of those generally known in the art. The catalysts employed in the examples were prepared according to the methods outlined in "The Fischer Tropsch and Related Syntheses" by H. H. Storch, N. Golumbic and R. B. Anderson, (John Wiley and Sons, Inc., 1951) the contents of which are incorporated herein by reference.

The ratio of zeolite to Fischer-Tropsch component may be from 0.1 to 50:1, preferably between 1 to 10:1. When the two components are combined as an intimate mixture, the powders of the components (greater than 30 mesh) are combined and thoroughly mixed in the desired ratio and a lubricant such as graphite can be added to the mix. One to two weight percent based upon the total catalyst weight is also sufficient. The mixture is then pressed into pellets or wetted and extruded, followed by drying and calcination. Pelleted catalysts are broken into 10-30 mesh particles before placement into the reactor.

The catalyst is finally placed in the reactor and the Fischer-Tropsch component activated in a known manner such as by reduction with hydrogen, carbon monoxide or a combination with or without the presence of a diluent such as nitrogen. Reduction conditions are chosen to optimize the activity of the particular Fischer-Tropsch component present in the catalyst. They normally include heating to a temperature of at least 100° C up to about 600° C at $H_2$ and/or CO partial pressures ranging from 0.001 MPa to about 5.07 MPa and for a period of time of from about one to 100 hours.

In the process of the present invention, synthesis gas, _i.e._, a mixture of hydrogen and carbon monoxide, in a ratio of 0.1 to 10:1 $H_2$/CO and preferably 0.5 to 4:1 $H_2$/CO, is reacted in the presence of the novel catalyst in the vapor phase to form principally highly branched liquid paraffins. The conversion can be conducted in either fluid bed or fixed bed reactors, the latter being exemplified herein, and the process is applicable to continuous or batch type operation.

The reaction temperature should be maintained between temperatures of from about 100° to 500° C, preferably from about 150° to 350° C. Pressures may be from ambient to about 200 atmospheres (20 MPa), preferably from ambient to about 30 atmospheres (3 MPa). Synthesis gas space velocities can range from about 10 to 100,000 GHSV, or volumes of gas passed over one equivalent volume of catalyst per hour, and preferably from about 100 to 10,000 GHSV.

Products of the synthesis gas conversion resulting from practice of the present invention include highly branched liquid paraffins, the majority of which boil below 204° C, the cut-off for good gasoline grade fractions. No measurable olefins or oxygenates are produced. Such liquids are useful as a blending stock for gasoline.

In the examples reported hereinbelow, a catalyst of the present invention was prepared in a two-step process as follows:

### Step I

Two grams of $[(NH_3)_4Pd]Cl_2$ was dissolved in about 300 ml of water. The solution was added to 100 gms of Linde zeolite Y-72 (LZY-72) $Na^+$ form powder was added to the first solution and the resulting mixture was heated to about 60° C and stirred for four hours. The solution was cooled to room temperature and stirred overnight. The zeolite was then filtered and was combined with 2.0 gms of $[(NH_3)_4Pd]Cl_2$ in 300 ml of water. The new solution was stirred for 24 hours after which the zeolite was again filtered. One gram of additional $[(NH_3)_4Pd]Cl_2$ was

dissolved in 200 ml of water, the zeolite was added and stirred for four hours. The zeolite was again filtered, washed with 500 ml of water and dried on the frit. The zeolite was then dried and calcined at 500° C for four hours. The modified zeolite contained 2.0 weight percent Pd and has been designated Pd-LZY-72 in the work which follows.

A similar modified zeolite was prepared according to the procedure set forth in Step I but with 100 gms of Linde zeolite Y-82 (LZY-82) $NH_4^+$ form powder in place of LZY-72. The modified zeolite obtained contained 2.3 weight percent Pd and has been designated Pd-LZY-82 in work hereinbelow.

Step II

Two parts of the calcined Pd-LZY-72 powder thus prepared, was combined with one part of a Fischer-Tropsch catalyst comprising $100Co:8MgO:5ThO_2:100$ kieselguhr. The solids were thoroughly mixed together and with 2 weight percent graphite. The mixture was pelleted, broken into 10 to 30 mesh particles and calcined at 500° C for four hours. The catalyst formed is one of the present invention and is designated as Catalyst A.

Two parts of the calcined Pd-LZY-82 powder was also combined with one part of the Fischer-Tropsch catalyst comprising $100Co:8MgO:5ThO_2:100$ kieselguhr. The solids were thoroughly mixed together and with 2 weight percent graphite. The mixture was pelleted, broken into 10 to 30 mesh particles and calcined at 500° C for four hours. The catalyst formed is one of the present invention and is designated as Catalyst B.

Catalyst A was reduced and then run in a fixed bed reactor for the conversion of synthesis gas as follows: 20 cc of Catalyst A was placed in a fixed bed reactor and pressurized to 0.7 MPa. 300 SCCM (standard cubic centimeters) of $H_2$ was passed over the catalyst while it was heated to 400° C, where the temperature was held constant for 17 hours, after which the reactor was cooled.

The bath temperature was set to 225° C and the pressure was increased to 1.04 MPa. Flows of 50 SCCM CO and 50 SCCM $H_2$ were introduced and a 151 minute run was conducted. A volume contraction of 54.6 percent was observed and 0.24 grams of organic liquid was produced per hour. Gas chromatographic analysis of the liquid showed it to be highly branched. The $C_{10}$ component, for example, was greater than 3:1 branched to straight chain. NMR analysis showed no functional groups to be present indicating a completely saturated product. The $CH_2/CH_3$ ratio of the organic was 1.6.

Next, flows of 100 SCCM $H_2$ with 50 SCCM CO were passed over the catalyst. The bath temperature was lowered to 216° C and a 1058 minute run was conducted. A gas contraction of 37.2 percent was observed and 0.29 gms of organic liquid was produced per hour. Gas chromatography again showed the sample to be highly branched, and NMR showed the presence of only saturated products. The $CH_2/CH_3$ ratio observed was 2.2. A simulated distillation analysis of the organic resulted in an average boiling point of 159° C, while 74 percent of the liquid boiled below 204° C, the cut off for the gasoline boiling range.

Additional synthesis gas conversions were run over the catalyst of the present invention in the manner just set forth and the results of all conversion are reported in the tables which appear hereinbelow.

For purposes of comparison with the catalysts of the present invention, three catalysts not part of the present invention and designated as C, D and E were also prepared by combining an unmodified zeolite with a Fischer-Tropsch catalyst following the procedure of Step II hereinabove. The description of the preparation of each is as follows:

### Catalyst C

1 part (LZY-82 ($NH_4^+$ analog of LZY-72) which had been calcined to give the $H^+$ form and 1 part of 100Co: $5ThO_2:8MgO:200$ kieselguhr were mixed and pelleted using the same procedure as Step II.

### Catalyst D

3 parts LZY-82 (calcined) and 1 part 100Co:
$5ThO_2:8MgO:200$ kieselguhr were mixed and pelleted as in
Step II.

### Catalyst E

1 part HZSM-5 ($SiO_2/Al_2O_3 \approx 70$) and 1 part 100Co:
$5ThO_2:8MgO:100$ kieselguhr were mixed and pelleted as in
Step II.

Catalysts C-E were also reduced and then run in a
fixed bed reactor for the conversion of synthesis gas as
follows: 20 cc of Catalyst C was placed in the reactor and
pressurized to 0.7 MPa with $H_2$. 300 SCCM of $H_2$ was intro-
duced and the system was heated to 400° C and maintained
for 17 hours. The pressure was reduced to ambient and
flows of 50 SCCM $H_2$ and 50 SCCM CO were fed at a bath
temperature of 225° C. A run was conducted for 1178
minutes and a contraction of 29.4 percent was observed with
production of 0.07 grams of liquid organic product per
hour. The liquid contained olefins in the amount of
$34.8 \times 10^{-4}$ moles per gram and aromatics at $2.1 \times 10^{-4}$ moles
per gram. The $CH_2/CH_3$ ratio of the organic liquid was 2.6.

20 cc of Catalyst D was placed in the reactor and
pressurized to 0.7 MPa with $H_2$. 300 SCCM of $H_2$ was intro-
duced and the system was heated to 400° C and maintained
for 16 hours. The pressure was increased to 1.04 MPa and
flows of 50 SCCM of $H_2$ and 50 SCCM of CO at a bath temperature of 240° C. A run was conducted for 1172 minutes
and a contraction of 65 percent was observed with production of 0.5 grams of liquid organic product per hour.
The liquid contained olefins in the amount of $26.0 \times 10^{-4}$
moles per gram and aromatics in the amount of $0.95 \times 10^{-4}$
moles per gram. The $CH_2/CH_3$ ratio observed was 2.5 and the
boiling point average from simulated distillation was 173°
C with 66 percent boiling below 204° C.

20 cc of Catalyst E was placed in the reactor and
pressurized to 0.7 MPa with $H_2$. 300 SCCM of $H_2$ was intro-
duced and the system was heated to 400° C and maintained overnight (about 16 hours). The pressure was increased to 1.04 MPa and flows of 50 SCCM $H_2$ and 50 SCCM CO were introduced at a bath temperature of 211° C. A run was conducted for 969 minutes with 51.1 percent contraction observed, with the production of 0.37 grams of organic liquid produced per hour. The liquid contained $47 \times 10^{-4}$ moles per gram of olefins and the average boiling point was 182° C and 62 percent of the organic boiled below 204° C, with a $CH_2/CH_3$ ratio in the sample of 3.1.

The $H_2$ flow was then increased to 100 SCCM over Catalyst E and the oven temperature was cut back to 204° C and a run of 1405 minutes conducted. The observed gas contraction was 67.7 percent and 0.35 grams of organic liquid was produced per hour. The product contained $9.9 \times 10^{-4}$ moles per gram of olefins. The observed $CH_2/CH_3$ ratio was 3.7 and the average boiling point was 181° C and 61 percent of the product boiled below 204° C. As the foregoing examples show, Catalyst A produced the lowest boiling product, the most branching and the least amount of undesirable products.

A separate catalyst apart from the invention was also prepared for comparison with Catalysts A and B of the present invention, wherein Pd was co-precipitated with the other component metals of the Fischer-Tropsch catalyst. This catalyst is designated as Catalyst F and was prepared in a similar fashion as the Co Fischer-Tropsch catalysts set forth hereinabove but with the addition of $Pd(NO_3)_2$ to the solution of metal salts. More particularly, the method of preparation of the Co Fischer-Tropsch catalysts presented herein has been set forth in Storch, et al _Id_. The modification of the Fischer-Tropsch catalysts was made by adding the appropriate amount of $Pd(NO_3)_2$ to the metal nitrate solution which was then co-precipitated with the other metals. The modified Fischer-Tropsch catalyst was then combined with 50 percent LZY-82 following the procedure otherwise set forth in Step II.

Catalysts A and B were next compared further against Catalysts C, D and F as well as three conventional Fischer-Tropsch catalyst compositions which did not contain any zeolite. The comparison catalysts included the following: Cobalt Fischer-Tropsch catalysts alone over different supports; Cobalt Fischer-Tropsch catalyst with zeolite, 50/50 mixture (Catalyst C); Cobalt Fischer-Tropsch catalyst with zeolite, 25/75 mixture (Catalyst D); Cobalt Fischer-Tropsch catalyst containing palladium with zeolite, 50/50 mixture (Catalyst F); Cobalt Fischer-Tropsch catalyst with palladium modified zeolite, 33.3/66.7 mixture (LZY-72-Pd Catalyst A and LZY-82-Pd Catalyst B). More specifically, the catalysts employed were as follows:

<u>Examples No. 1-6</u>

$100Co:5ThO_2:8MgO:200Kieselguhr$ on alundum

<u>Examples No. 7-8</u>

$100Co:5ThO_2:8MgO:200Kieselguhr(10cc)$ + 10cc-glass beads

<u>Examples No. 9-11</u>

Catalyst C:  $50\%(100Co:5ThO_2:8MgO:200Kieselguhr)/50\%LZY-82$

<u>Examples No. 12-16</u>

Catalyst D:  $25\%(100Co:5ThO_2:8MgO:200Kieselguhr)/75\%LZY-82$

<u>Examples No. 17-20</u>

Catalyst F:  $50\%(100Co:10MgO:5ThO_2:2Pd:100Kieselguhr)/50\%LZY-8$

<u>Examples No. 21-23</u>

Catalyst A:  $33.3\%(100Co:8MgO:5ThO_2:100Kieselguhr)/$
$66.7\%LZY-72-Pd$

<u>Examples No. 24-27</u>

Catalyst B:  $33.3\%(100Co:8MgO:5ThO_2:100Kieselguhr)/$
$66.7\%LZY-82-Pd$

In each series of examples, 20 cc of a specific catalyst was selected, placed in the reactor after appropriate reduction of its Fischer-Tropsch component and then subjected to multiple runs with increasing time being reported in Table II.

The Fischer-Tropsch catalysts were first reduced at ambient pressure or greater under gases at temperatures and for times as set forth in Table I. Reductions of the Fischer-Tropsch components of Catalysts A-D and F appear in Table II for the first numbered example following each new catalyst.

TABLE I

Reduction Conditions for Fischer-Tropsch Catalysts

| Ex. No. | T °C | P MPa gauge | Gas | Time |
|---|---|---|---|---|
| 1 | 350 | 1.04 | $H_2$ | 18 |
| 2 | 350 | 1.04 | $H_2$ | 18 |
| 3 | 350 | Amb. | $H_2$ | 4 |
| 4 | 350 | 1.04 | $H_2$ | 16 |
| 5 | 350 | 1.04 | $H_2$ | 16 |
| 6 | 350 | 1.04 | $H_2$ | 16 |
| 7 | 400 | Amb. | $3:1H_2/N_2$ | 17 |
| 8 | 400 | Amb. | $3:1H_2/N_2$ | 17 |

Reactions of synthesis gas over the catalysts were conducted under conditions set forth in Table II which reports grams of organic product and water produced per hour. Gas contraction reported in Table II is determined as follows: [1-(volume gas out/volume gas in)]X100. Feed of synthesis gas is given as SCCM (standard cubic centimeters). Analysis of the liquid products by NMR was conducted and has been reported in Table III. The amounts present equal $1 \times 10^{-4}$ moles/gm of liquid. Lastly, Table IV presents per pass conversion and percent selectivity. Calculations of the percent of CO converted to a particular product (%ppc) and percent selectivity (% sel) for the gaseous products of Examples 9, 11, 14-16, 18-20, 22, 23 and 25-27 were determined as follows:

% ppc = [moles of CO in product/moles of CO fed] X 100 % sel = [SCCM product/SCCM CO (converted)] X 100

## TABLE II

### Synthesis Gas Conversion Process

| Ex. No. | T Bed | T exo | P gauge | GHSV | $CO/H_2$ | Contraction | Time | Total Liquid Organic Product (gms/hr) |
|---|---|---|---|---|---|---|---|---|
| Fischer-Tropsch Catalysts Alone | | | | | | | | |
| 1 | 180 | 185 | 1.04 | 175 | 58/59 | 7% | - | - |
| 2 | 202 | 209 | 1.04 | 175 | 57/59 | 59% | 2 hr. | 0.20 |
| 3 | 196 | 202 | 1.04 | 168 | 54/58 | 45% | 2 hr. | 0.14 |
| 4 | 195 | 202 | 1.04 | 250 | 53/114 | 40% | 2 hr. | 0.43 |
| 5 | 256 | 268 | 1.04 | 210 | 65/75 | 70% | 3 hr. | 0.66 |
| 6 | 257 | 276 | 1.04 | 300 | 65/136 | 78% | 2 hr. | 1.00 |
| 7 | 224 | 233 | 1.04 | 1200 | 98/98 | ~25% | 2 hr. 2 min. | 0.25 |
| 8 | 224 | 230 | 1.04 | 1200 | 101/101 | 20% | 20 hr. | 0.16 |
| Catalyst C | | | | | | | | |
| 9 | -- | -- | 0.7 | 300 | 0/300 | - | 17 hr. | - |
| 10 | 219 | 227 | 4.2 | 600 | 100/100 | 49.2% | 3 hr. 49 min. | 0 |
| 11 | 225 | 234 | 0 | 300 | 50/50 | 29.4% | 19 hr. 38 min. | 0.07 |
| Catalyst D | | | | | | | | |
| 12 | -- | -- | 0.7 | 900 | 0/300 | - | 16 hr. | - |
| 13 | 241 | 251 | 1.04 | 300 | 50/50 | 30.1% | 2 hr. 28 min. | 0 |
| 14 | 240 | 247 | 0 | 300 | 50/50 | 21.6% | 16 hr. 29 min. | 0 |
| 15 | 240 | 258 | 1.04 | 300 | 50/50 | 65.7% | 4 hr. 23 min. | 0.46 |
| 16 | 240 | 260 | 1.04 | 300 | 50/50 | 64.9% | 19 hr. 32 min. | 0.50 |

TABLE II (Continued)

Synthesis Gas Conversion Process

| Ex. No. | T Bed | T exo | P gauge | GHSV | CO/H$_2$ | Contraction | Time | Total Liquid Organic Product (gms/hr) |
|---------|-------|-------|---------|------|----------|-------------|------|----------------------------------------|
| Catalyst F | | | | | | | | |
| 17 | – | – | 1.04 | 900 | 0/300 | – | 23 hr. | – |
| 18 | 185 | 230 | 1.04 | 300 | 50/50 | 40.7% | 3 hr. 32 min. | 0.4 |
| 19 | 205 | 217 | 1.04 | 450 | 50/100 | 72.3% | 15 hr. 53 min. | 0.69 |
| 20 | 215 | 224 | 1.04 | 300 | 50/50 | 54.7% | 3 hr. 46 min. | 0.45 |
| Catalyst A | | | | | | | | |
| 21 | – | – | 0.7 | 900 | 0/300 | – | 17 hr. | – |
| 22 | 225 | 239 | 1.04 | 300 | 50/50 | 54.6% | 2 hr. 31 min. | 0.24 |
| 23 | 216 | 224 | 1.04 | 450 | 50/100 | 37.2% | 17 hr. 38 min. | 0.29 |
| Catalyst B | | | | | | | | |
| 24 | – | – | 0.7 | 900 | 0/300 | – | 20 hr. 30 min. | – |
| 25 | 220 | 234 | 1.04 | 300 | 50/50 | 51.1% | 18 hr. 5 min. | 0.39 |
| 26 | 220 | 234 | 1.04 | 450 | 50/100 | 48.4% | 4 hr. 38 min. | 0.43 |
| 27 | 220 | 235 | 1.04 | 450 | 50/100 | 62.3% | 15 hr. 30 min. | 0.64 |

-14-

## TABLE III

### Liquid Products From Synthesis Gas Conversion

| Ex. No. | SIMDIS °C 26% | SIMDIS °C 50% | SIMDIS °C 96% | VOL. AVG B.P. °C | % < 204° | $CH_2/CH_3$ | Vinyl Olefin | Internal Olefin | Vinylidene Olefin | Trisub Olefin | Olefin Total | Alc. | Acid | Ar. |
|---|---|---|---|---|---|---|---|---|---|---|---|---|---|---|
| **Fischer-Tropsch** | | | | | | | | | | | | | | |
| **Catalyst Alone** | | | | | | | | | | | | | | |
| 1 | – | – | – | – | – | – | 14.4 | 4.34 | – | – | – | 1.58 | 2.18 | – |
| 7 | 189 | 252 | 404 | 254 | 33 | 6.5 | 18.7 | 6.2 | – | – | – | 10.1 | – | – |
| **Catalyst C** | | | | | | | | | | | | | | |
| 11 | – | – | – | – | – | 2.6 | .92 | 19.9 | 0.4 | 13.6 | 34.8 | 0 | 0 | 2.1 |
| **Catalyst D** | | | | | | | | | | | | | | |
| 15 | – | – | – | – | – | 2.3 | 2.4 | 20.3 | 1.0 | 13.4 | 37.1 | 0 | 0 | 1.8 |
| 16 | 101 | 162 | 355 | 173 | 66 | 2.5 | .95 | 9.7 | .99 | 14.4 | 26.0 | 0 | 0 | .95 |
| **Catalyst F** | | | | | | | | | | | | | | |
| 18 | – | – | – | – | – | 4.0 | 0 | 0 | 7.5 | 0 | 7.5 | 0 | 0 | 0 |
| 19 | 90 | 149 | 337 | 159 | 67 | 2.8 | 0 | 11.9 | 0 | 0 | 11.9 | 0 | 0 | 0 |
| 20 | 103 | 173 | 360 | 180 | 62 | 3.0 | 0 | 26.3 | 0 | 0 | 26.3 | 0 | 0 | 0 |
| **Catalyst A** | | | | | | | | | | | | | | |
| 21 | – | – | – | – | – | 1.6 | 0 | 0 | 0 | 0 | 0 | 0 | 0 | 0 |
| 22 | 113 | 151 | 312 | 159 | 74 | 2.2 | 0 | 0 | 0 | 0 | 0 | 0 | 0 | 0 |

TABLE III (Continued)

Liquid Products From Synthesis Gas Conversion

| Ex. No. | SIMDIS °C 26% | 50% | 96% | VOL. AVG B.P. °C | % < 204° | CH₂/CH₃ | Vinyl Olefin | Internal Olefin | Vinylidene Olefin | Trisub Olefin | Olefin Total | Alc. | Acid | Ar. |
|---|---|---|---|---|---|---|---|---|---|---|---|---|---|---|
| Catalyst B | | | | | | | | | | | | | | |
| 25 | 73 | 110 | 276 | 121 | 86 | 1.3 | 0 | 0 | 0 | 0 | 0 | 0 | 0 | 0 |
| 26 | 82 | 118 | 299 | 130 | 84 | 1.7 | 0 | 0 | 0 | 0 | 0 | 0 | 0 | 0 |
| 27 | 60 | 105 | 291 | 116 | 85 | 2.0 | 0 | 0 | 0 | 0 | 0 | 0 | 0 | 0 |

0153517

# TABLE IV

## Conversion of CO and Selectivity of Catalysts A, B, C, D and F

| Ex. No. | 9 - Catalyst C | | | 11 - Catalyst C | | | 14 - Catalyst D | | | 15 - Catalyst D | | |
|---|---|---|---|---|---|---|---|---|---|---|---|---|
| | SCCM | %PPC | %SEL | SCCM | %PPC | %SEL | SCCM | %PPC | %SEL | SCCM | %PPC | %SEL |
| **Products** | | | | | | | | | | | | |
| CO (conv) | 8.36 | – | – | 11.53 | – | – | 9.08 | – | – | 25.96 | – | – |
| CO | 91.64 | – | – | 38.47 | – | – | 40.92 | – | – | 24.04 | – | – |
| $CO_2$ | 0.45 | 0.90 | 5.38 | 0.30 | 0.60 | 2.60 | 0.32 | 0.64 | 3.52 | 1.89 | 3.78 | 7.28 |
| $CH_4$ | 0.28 | 0.56 | 3.35 | 1.77 | 3.54 | 15.35 | 1.41 | 2.82 | 15.53 | 1.65 | 3.30 | 6.36 |
| $C_2H_6$ | – | – | – | – | – | – | – | – | – | – | – | – |
| $C_2H_4^=$ | – | – | – | – | – | – | – | – | – | – | – | – |
| $C_3H_8$ | 0.15 | 0.30 | 1.79 | 0.23 | 0.46 | 2.00 | 0.23 | 0.46 | 2.53 | 0.45 | 0.90 | 1.73 |
| $C_3H_6^=$ | 0.12 | 0.24 | 1.44 | 0.93 | 1.80 | 8.07 | 0.79 | 1.58 | 8.70 | 0.68 | 1.36 | 2.62 |
| $i\text{-}C_4H_{10}$ | 0.16 | 0.32 | 1.91 | 0.03 | 0.06 | 0.26 | 0.06 | 0.12 | 0.66 | 0.18 | 0.36 | 0.69 |
| $nC_4H_{10}$ | 0.16 | 0.32 | 1.91 | 0.23 | 0.46 | 2.00 | 0.16 | 0.32 | 1.76 | 0.29 | 0.58 | 1.12 |
| $1\text{-}C_4H_8^=$ | – | – | – | 0.31 | 0.62 | 2.69 | 0.16 | 0.32 | 1.76 | 0.14 | 0.28 | 0.54 |
| $i\text{-}C_4H_8^=$ | – | – | – | 0.06 | 0.12 | 0.52 | 0.09 | 0.18 | 0.99 | 0.03 | 0.06 | 0.12 |
| $t\text{-}C_4H_8^=$ | – | – | – | 0.40 | 0.80 | 3.47 | 0.47 | 0.94 | 5.10 | 0.44 | 0.88 | 1.70 |
| $c\text{-}C_4H_8^=$ | – | – | – | 0.28 | 0.56 | 2.43 | 0.28 | 0.56 | 3.08 | 0.26 | 0.52 | 1.00 |
| $i\text{-}C_5H_{12}$ | 0.30 | 0.60 | 3.59 | – | – | – | 0.16 | 0.32 | 1.76 | 0.29 | 0.58 | 1.12 |
| $nC_5H_{12}$ | – | – | – | – | – | – | – | – | – | 0.15 | 0.30 | 0.58 |
| Liq. gas equiv. | – | – | – | – | 3.78 | 16.40 | – | – | – | – | 24.70 | 47.57 |

TABLE IV (Continued)

Conversion of CO and Selectivity of Catalysts A, B, C, D and F

| Ex. No. | 16 – Catalyst D | | | 18 – Catalyst F | | | 19 – Catalyst F | | | 20 – Catalyst F | | |
|---|---|---|---|---|---|---|---|---|---|---|---|---|
| | SCCM | %PPC | %SEL | SCCM | %PPC | %SEL | SCCM | %PPC | %SEL | SCCM | %PPC | %SEL |
| Products | | | | | | | | | | | | |
| CO (conv) | 22.73 | – | – | 27.2 | – | – | 39.2 | – | – | 21.3 | – | – |
| CO | 27.27 | – | – | 22.8 | 45.7 | – | 10.8 | 21.7 | – | 28.7 | 57.4 | – |
| $CO_2$ | 1.72 | 3.44 | 7.57 | 3.8 | 7.6 | 14.0 | .38 | .75 | 1.0 | .47 | .94 | 2.2 |
| $CH_4$ | 2.25 | 4.50 | 9.90 | 3.3 | 6.6 | 12.2 | 5.9 | 11.9 | 15.2 | 1.8 | 3.7 | 8.7 |
| $C_2H_6$ | 0.77 | 1.54 | 3.39 | 0.6 | 1.2 | 2.1 | 1.4 | 2.7 | 3.4 | 0.8 | 1.6 | 3.8 |
| $C_2H_4^=$ | 0.08 | 0.16 | 0.35 | – | – | – | – | – | – | – | – | – |
| $C_3H_8$ | 0.46 | 0.92 | 2.02 | 0.55 | 1.1 | 2.1 | 2.1 | 4.3 | 5.5 | .85 | 1.7 | 4.0 |
| $C_3H_6^=$ | 0.80 | 1.60 | 3.52 | .095 | .19 | .3 | .36 | .72 | .9 | .55 | 1.1 | 2.6 |
| $i-C_4H_{10}$ | 0.07 | 0.14 | 0.31 | .65 | 1.3 | 2.4 | .09 | .19 | .2 | .04 | .08 | .19 |
| $nC_4H_{10}$ | 0.29 | 0.58 | 1.28 | .40 | .79 | 1.5 | 1.3 | 2.5 | 3.2 | .50 | 1.0 | 2.3 |
| $1-C_4H_8^=$ | 0.15 | 0.30 | 0.66 | – | – | – | .11 | .23 | .30 | .12 | .25 | 0.6 |
| $i-C_4H_8^=$ | 0.04 | 0.08 | 0.18 | – | – | – | .04 | .07 | .09 | .03 | .06 | .15 |
| $t-C_4H_8^=$ | 0.49 | 0.98 | 2.16 | .05 | .10 | .17 | .43 | .87 | 1.1 | .41 | .82 | 1.9 |
| $c-C_4H_8^=$ | 0.29 | 0.58 | 1.28 | .095 | .19 | .38 | .24 | .48 | .6 | .24 | .47 | 1.1 |
| $i-C_5H_{12}$ | 0.12 | 0.24 | 0.53 | .85 | 1.7 | 3.1 | – | – | – | – | – | – |
| $nC_5H_{12}$ | 0.18 | 0.36 | 0.79 | – | – | – | – | – | – | – | – | – |
| Liq. gas equiv. | – | 26.82 | 59.00 | – | 2.0 | 3.6 | – | 37.0 | 47.3 | – | 24.2 | 56.8 |

-18-

0153517

TABLE IV (Continued)

Conversion of CO and Selectivity of Catalysts A, B, C, D and F

| Ex. No. | 22 – Catalyst A | | | 23 – Catalyst A | | | 25 – Catalyst B | | |
|---|---|---|---|---|---|---|---|---|---|
| | SCCM | %PPC | %SEL | SCCM | %PPC | %SEL | SCCM | %PPC | %SEL |
| **Products** | | | | | | | | | |
| CO (conv) | 22.95 | – | – | 10.06 | – | – | 22.9 | – | – |
| CO | 27.05 | – | – | 39.94 | – | – | 27.1 | 54.2 | – |
| $CO_2$ | 1.68 | 3.36 | 7.32 | – | – | – | .21 | .42 | .91 |
| $CH_4$ | 1.63 | 3.26 | 7.10 | 3.27 | 6.54 | 19.68 | .95 | 1.9 | 4.1 |
| $C_2H_6$ | 0.82 | 1.64 | 3.57 | 1.21 | 2.42 | 7.28 | .6 | 1.2 | 2.6 |
| $C_2H_4^=$ | – | – | – | – | – | – | – | – | – |
| $C_3H_8$ | 1.36 | 2.72 | 5.92 | 2.03 | 4.06 | 12.21 | 1.2 | 2.4 | 5.3 |
| $C_3H_6^=$ | 0.20 | 0.40 | 0.87 | – | – | – | .02 | .04 | .09 |
| $i\text{-}C_4H_{10}$ | 0.69 | 1.38 | 3.01 | 0.20 | 0.40 | 1.20 | 0.9 | 1.8 | 4.0 |
| $nC_4H_{10}$ | 1.14 | 2.20 | 4.97 | 1.66 | 1.32 | 9.99 | 1.1 | 2.3 | 5.1 |
| $1\text{-}C_4H_8^=$ | – | – | – | – | – | – | – | – | – |
| $i\text{-}C_4H_8^=$ | – | – | – | – | – | – | – | – | – |
| $t\text{-}C_4H_8^=$ | 0.09 | 0.18 | 0.39 | – | – | – | – | – | – |
| $c\text{-}C_4H_8^=$ | 0.04 | 0.08 | 0.17 | – | – | – | – | – | – |
| $i\text{-}C_5H_{12}$ | 0.96 | 1.92 | 4.18 | 0.44 | 0.88 | 3.31 | 1.3 | 2.6 | 5.6 |
| $nC_5H_{12}$ | 0.30 | 0.60 | 1.31 | – | – | – | – | – | – |
| Liq. gas equiv. | – | 12.90 | 28.10 | – | 15.62 | 46.99 | – | 20.7 | 45.2 |

0153517

TABLE IV (Continued)

Conversion of CO and Selectivity of Catalysts A, B, C, D and F

| Ex. No. | 26 – Catalyst B | | | 27 – Catalyst B | | |
|---|---|---|---|---|---|---|
| | SCCM | %PPC | %SEL | SCCM | %PPC | %SEL |
| Products | | | | | | |
| CO (conv) | 26.3 | – | – | 34.0 | – | – |
| CO | 23.7 | 47.4 | – | 16.0 | 31.9 | – |
| $CO_2$ | .28 | .57 | 1.1 | .32 | .63 | .92 |
| $CH_4$ | 4.4 | 8.8 | 16.8 | 4.8 | 9.7 | 14.2 |
| $C_2H_6$ | 1.4 | 2.7 | 5.2 | 1.4 | 2.9 | 4.2 |
| $C_2H_4^=$ | – | – | – | – | – | – |
| $C_3H_8$ | 2.6 | 5.1 | 9.7 | 2.2 | 4.4 | 6.5 |
| $C_3H_6^=$ | – | – | – | – | – | – |
| $i-C_4H_{10}$ | 1.3 | 2.5 | 4.8 | .85 | 1.7 | 2.6 |
| $nC_4H_{10}$ | 2.2 | 4.5 | 8.5 | 1.8 | 3.6 | 5.3 |
| $1-C_4H_8^=$ | – | – | – | – | – | – |
| $i-C_4H_8^=$ | – | – | – | – | – | – |
| $t-C_4H_8^=$ | – | – | – | – | – | – |
| $c-C_4H_8^=$ | – | – | – | – | – | – |
| $i-C_5H_{12}$ | 1.5 | 3.0 | 5.7 | .85 | 1.7 | 2.5 |
| $nC_5H_{12}$ | – | – | – | – | – | – |
| Liq. gas equiv. | – | 22.9 | 43.5 | – | 34.3 | 50.3 |

With reference to the tables, it can be seen first from Table II, that the Fischer-Tropsch catalysts produced the greatest amount of organic materials and that several of the examples representing the invention reported organic product yield as good as or better than Catalysts C and D where Fischer-Tropsch compounds were combined with unmodified zeolite.

In Table III, it is seen that Catalysts A and B produced neither olefins nor oxygenates while Catalysts C, D and F as well as the Fischer-Tropsch catalysts were particularly high in olefins. Catalyst F, it will be recalled comprised palladium in the Fischer-Tropsch component rather than the zeolite and although no oxygenates were produced, the presence of olefins was significant. In this respect, Catalyst F was not different from Catalysts C and D (no palladium) demonstrating that the metal is not inherently effective but rather, its incorporation into the zeolite portion of the combination catalyst is.

It is also to be noted from Table III that the volume of organic liquids boiling below 204° C was greatest with Catalysts A and B and that the best (lowest) $CH_2/CH_3$ ratios were also obtained. The methylene to methyl ratio can be utilized to determine the chain length or extent of branching. For instance, comparing the ratio of a straight chain $C_{16}$ paraffin (7/1) to that for a $C_8$ paraffin (3.0/1) it appears that the longer chain has the higher number. However, when a $C_8$ paraffin is branched, such as iso-octane the ratio becomes 1/5 or 0.20, from which it is evident that branching lowers the ratio considerably.

By comparing the $CH_2/CH_3$ number with boiling point fraction data, i.e., SIMDIS (simulated distillation), volume average boiling point and percent boiling under 204° C, the usefulness of the product as a gasoline former or as gasoline can also be determined. Inasmuch as greater chain length increases the $CH_2/CH_3$ ratio while branching decreases it and longer chain compounds have higher boiling points, a ratio of about 2.5 coupled with a high percent of materials boiling under 204° C, is indicative of a good gasoline former while a value of 2.0 is excellent.

Finally, with respect to Table IV, it will be seen that Catalysts A and B generally produced from small amounts of olefins (Examples 22 and 25) to none (Examples 23, 26 and 27). Branching, on the other hand, was significant as exemplified by the $C_4$ and $C_5$ range of products.

Based upon the satisfactory yields of liquid paraffins, particularly branched species, the minimal amounts of olefins and lack of oxygenates that have been obtained when the catalysts of the present invention have been employed in the process set forth herein, it should be apparent that the objects of the invention have been met. It is to be understood that the catalysts of the present invention can be modified with other reducible metals from Groups VIII, IB or IIB than palladium. Similarly, other Fischer-Tropsch catalysts can be employed.

It should be apparent to those skilled in the art that the process of the subject invention is operable with catalysts having fairly broad ratios of modifier to zeolite and of zeolite to Fischer-Tropsch catalyst and that the process is operable when other temperatures, pressures and times are employed. Similarly, parameters for the preparation of the catalyst can be varied from those which have been exemplified. It is to be understood that these variables fall within the scope of the claimed invention and that the subject invention is not to be limited by the examples set forth herein. These have been provided merely to demonstrate operability and, therefore, the selection of specific catalyst components and process conditions can be determined without departing from the spirit of the invention herein disclosed and described. Moreover, the scope of the invention shall include all modifications and variations that may fall within the scope of the attached claims.

0153517

CLAIMS:

1.    A catalyst composition comprising:

a medium to large part zeolite modified by the metal salt of an element selected from the group consisting of Group VIII, IB and IIB elements;  and

a Fischer-Tropsch catalyst wherein the ratio of zeolite to Fischer-Tropsch component is from 0.1 to 50:1.

2.    A catalyst as claimed in claim 1 characterised in that the amount of the metal in the modified zeolite comprises from 0.01 to 20 percent by weight.

3.    A catalyst as claimed in claim 1 or claim 2 characterised in that the elements are preferably selected from Group VIII.

4.    A catalyst as claimed in claim 3 characterized in that the zeolite is modified with palladium in an amount of 2.0 percent by weight and two parts of said modified zeolite are combined with one part by weight of said Fischer-Tropsch catalyst.

5.    A catalyst as claimed in claim 4 characterised in that the zeolite comprises a sodium exchanged form of zeolite Y and the Fischer-Tropsch catalyst comprises $100Co:8MgO:5ThO_2:100$ kieselguhr.

6.    A catalyst as claimed in claim 3 characterised in that the zeolite is modified with palladium in an amount of 2.3 percent by weight and two parts of said modified zeolite are combined with one part by weight of said Fischer-Tropsch catalyst.

7.    A catalyst, as claimed in claim 6 characterised in that the zeolite comprises an ammonium exchanged form of zeolite Y and the Fischer-Tropsch catalyst comprises $100Co:8MgO:5ThO_2:100$ kieselguhr.

8.    A catalyst as claimed in claim 5 or claim 7 characterised in that it further comprises at least one percent by weight of a lubricant.

9.    A catalyst as claimed in claim 8 characterised in that the lubricant is graphite.

10. A process for producing fuel grade saturated gaseous hydrocarbons comprising the steps of:

contacting synthesis gas having a ratio of hydrogen to carbon oxides of from 0.1 to 10.0:1 over a catalyst composition at a reaction temperature of from 100°C to 500°C and at a pressure of from 0.1 MPa to 20 MPa and at a gas hourly space velocity of from 10 to 100,000, characterised in that the catalyst composition is as claimed in any of claims 1 to 9.

11. A process as claimed in claim 10 characterised in that the synthesis gas composition is 2:1 $H_2$/CO.

12. A process as claimed in claim 11 characterised in that the temperature is 220°C, the pressure is 1.04 MPa and the GHSV is 450.

13. A process as claimed in claim 10 characterised in that the synthesis gas composition is 1:1 $H_2$/CO.

14. A process as claimed in claim 13 characterised in that the temperature is 220°C, the pressure is 1.04 MPa andthe GHSV is 300.

15. A process as claimed in any of claims 10 to 14 characterised in that it includes the further step of:

reducing said combination catalyst with $H_2$ and/or CO at a temperature of from 100°C to 600°C, at $H_2$ and/or CO partial pressures within the range of 0.001 MPa to 5.07 MPa and for a period of time of from one to 100 hours prior to said step of contacting.

16. A method for the preparation of a combination catalyst as claimed in any of claims 1 to 9 characterised in that one modifies a medium to large port zeolite with a metal salt of an element selected from the group consisting of Group VIII, IB and IIB; and

combines the modified zeolite with a Fischer-Tropsch catalyst in a ratio of from 0.1 to 50:1.

17. A method as claimed in claim 16 characterised in that it includes in a further step combining at least one percent by weight of a lubricant with the modified zeolite and the Fischer-Tropsch catalyst, which lubricant is preferably graphite.

18.   A method as claimed in claim 16 or claim 17
characterised in that it comprises the further step of:
      calcining the modified zeolite at a temperature
within the range of 100° to 800°C for at least one hour
prior to the step of combining.

19.   A method as claimed in claim 18 characterised in
that it includes the further step of reducing said
combination catalyst with $H_2$ and/or CO at a temperature
of from 100°C to 600°C, at $H_2$ and/or CO partial pressures
ranging from 0.001 MPa to 5.07 MPa and for a period of
time of from one to 100 hours prior to employing said
catalyst for the conversion of synthesis gas.

**0153517**

European Patent
Office

**EUROPEAN SEARCH REPORT**

. Application number

EP 84 30 1344

## DOCUMENTS CONSIDERED TO BE RELEVANT

| Category | Citation of document with indication. where appropriate, of relevant passages | Relevant to claim | CLASSIFICATION OF THE APPLICATION (Int. Cl 4) |
|---|---|---|---|
| X | GB-A-2 099 716 (RESEARCH ASSOCIATION)<br><br>* page 2, lines 58-62; page 2, lines 43-65; page 3, lines 1-9, 16-24 * | 1-4,6, 10-14, 16,18 | C 07 C 1/04<br>B 01 J 29/06 |
| Y | | 1,8-10, 17 | |
| A | | 5,7 | |
| Y,D | FR-A-2 268 771 (MOBIL OIL)<br><br>* page 2, lines 23-33; page 5, lines 11-23, 33-34; page 9, lines 16-20; page 11, lines 24-30; page 13, lines 33-34 * | 1,8-10, 17 | |
| A | | 5,7 | TECHNICAL FIELDS SEARCHED (Int. Cl.4) |
| A | US-A-4 399 234 (GULF)<br><br>* column 7, lines 1-3, 45-52 * | 5,7,15, 19 | C 07 C<br>B 01 J |
| A | EP-A-0 099 715 (BRITISH PETROLEUM)<br>* page 2, lines 17-21; page 3, lines 15-17 * | 5,7 | |

--- -/-

The present search report has been drawn up for all claims

| Place of search | Date of completion of the search | Examiner |
|---|---|---|
| THE HAGUE | 02-11-1984 | SALA P.C. |

European Patent
Office

**EUROPEAN SEARCH REPORT**

0153517

Application number

EP   84 30 1344

Page   2

## DOCUMENTS CONSIDERED TO BE RELEVANT

| Category | Citation of document with indication. where appropriate. of relevant passages | Relevant to claim | CLASSIFICATION OF THE APPLICATION (Int Cl 4) |
|---|---|---|---|
| A | CHEMICAL ABSTRACTS, vol. 78, no. 12, 26th March 1973, page 135, no. 74437e, Columbus, Ohio, US; T.F. BULANOVA et al.: "Cobalt-zirconia catalysts for the synthesis of hydrocarbons from carbon monoxide and hydrogen" & ZH. PRIKL. KHIM. (LENINGRAD) 1972, 45(10), 2300-4 ----- | 5,7 | |
| | | | TECHNICAL FIELDS SEARCHED (Int. Cl 4) |

The present search report has been drawn up for all claims

| Place of search THE HAGUE | Date of completion of the search 02-11-1984 | Examiner SALA P.C. |
|---|---|---|